# EUROPEAN PATENT SPECIFICATION

(11) **EP 4 256 072 B1**
(45) Date of publication and mention of the grant of the patent: **18.11.2026**
(21) Application number: 21901395.0
(22) Date of filing: 01.12.2021
(51) Int. Cl.: G01N 33/576, G01N 33/53

(54) **LITHIUM HEPARIN AS A BLOCKING AGENT**
LITHIUMHEPARIN ALS BLOCKER
HÉPARINE DE LITHIUM UTILISÉE EN TANT QU'AGENT DE BLOCAGE

(30) Priority: 03.12.2020 US 202063121161 P
(43) Date of publication of application: 11.10.2023
(73) Proprietor: Ortho-Clinical Diagnostics, Inc., Raritan, NJ 08869 (US)
(72) Inventor: HIRSCH, Brett, Raritan, NJ 08869 (US); CONTESTABLE, Paul, Raritan, NJ 08869 (US)
(74) Representative: Murgitroyd & Company
(86) International application number: PCT/US2021/061431
(87) International publication number: WO 2022/119937

(56) References cited:
- WO-A1-2019/183627
- US-A1- 2011 183 364
- US-A1- 2011 287 468
- US-A1- 2015 079 612
- US-A1- 2019 086 412
- US-A1- 2020 300 873
- HOWARD JUDITH ET AL: "Comparison of paired serum and lithium heparin plasma samples for the measurement of serum amyloid A in horses using an automated turbidimetric immunoassay", VETERINARY JOURNAL, vol. 199, no. 3, 1 March 2014 (2014-03-01), GB, pages 457 - 460, XP093190371, ISSN: 1090-0233, DOI: 10.1016/j.tvjl.2013.12.007
- D'ANGELO RYAN G., MICHAEL KLEPSER, RYAN WOODFIELD, HEMINA PATEL: "Hepatitis C Virus Screening: A Review of the OraQuick Hepatitis C Virus Rapid Antibody Test", JOURNAL OF PHARMACY TECHNOLOGY, vol. 31, no. 1, 1 January 2015 (2015-01-01), pages 13 - 19, XP055941702, Retrieved from the Internet <URL:https://www.ncbi.nlm.nih.gov/pmc/articles/PMC5990168/pdf/10.1177_8755122514548228.pdf> [retrieved on 20220712], DOI: 10.1177/8755122514548228

## Description

### FIELD

Described herein are blocking agents used in immunodiagnostic assays, related devices and systems, and methods of using the same. The invention relates to the use of lithium heparin as a blocking reagent to reduce a rate of false positive results in an immunoassay for detection of hepatitis C antibodies in a blood sample. Devices, systems and methods are described as they are useful for understanding the use of the invention.

### SUMMARY

Described herein are blocking agents that can be used in diagnostic assays. In some embodiments, the diagnostic assays can be immunoassays. US 2011/0183364 relates to an analytical method for the detection of latent hepatitis C. The invention provides use of lithium heparin as a blocking reagent to reduce a rate of false positive results in an immunoassay for detection of hepatitis C antibodies in a blood sample. Optional features of the invention are listed in the dependent claims. Further subject matter in this summary and the following detailed description is described to provide further understanding of the invention and of the technical background of the art.

The blocking agents described reduce a rate of false positive results detected in a patient population.

The blocking agent can be added to a reagent mixture or added as a reagent during an assay.

A blocking agent can include heparin. A blocking agent can include lithium. In the invention, the blocking agent is lithium heparin.

Immunoassay reagents may include one or more analyte binding agents in a diluent, and a blocking agent in an amount sufficient to reduce non-specific binding in an assay of a sample for the analyte.

Further disclosed but not claimed is a sample composition which comprises a sample to be assayed for the presence of an analyte, an analyte binding agent, and a blocking agent in an amount sufficient to reduce non-specific binding in an assay of the sample for the analyte.

Also disclosed but not claimed are methods of detecting an analyte in a sample, using a blocking agent to reduce non-specific binding. The methods comprise combining a sample to be analyzed for the presence of an analyte with a blocking agent and one or more analyte binding agents, so as to form a complex of any analyte present in the sample and the analyte binding agent(s), wherein the blocking agent reduces non-specific binding, and detecting the resulting complex so as to detect the analyte.

### BRIEF DESCRIPTION OF THE DRAWINGS

FIG. 1 is a general flow chart of an assay as described herein.
FIG. 2 is a graph showing assays run with and without lithium heparin.

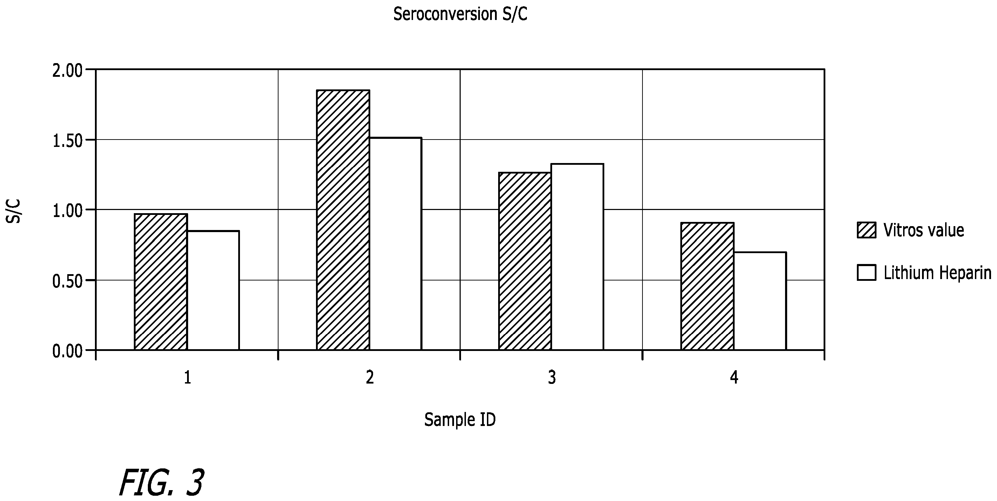

FIG. 3 is a graph showing retention of classification of samples that include lithium heparin.

### DETAILED DESCRIPTION

Described herein are blocking agents that can be used in diagnostic assays to prevent false positive results. The diagnostic assays of the invention are immunoassays. Compositions and systems disclosed herein include an immunoassay reagent which may comprise one or more analyte binding agent(s) in a diluent, and a blocking agent in an amount sufficient to reduce non-specific binding in an assay of a sample for the analyte.

The diagnostic assays and/or immunoassays described herein can be performed in any manner that achieves a result. The diagnostic assays and/or immunoassays can be performed as a benchtop assay. The diagnostic assays and/or immunoassays can be performed in an automated or semi-automated system. The diagnostic assays and/or immunoassays can be run on a mainframe analyzer. The diagnostic assays and/or immunoassays can be in vitro tests.

It is often desirable to determine the presence and the concentration of analytes in biological specimens or samples. An analyte is a substance or chemical constituent that is determined in an analytical procedure (such as an immunoassay). Immunoassays are based on the concept of binding partners. An analyte of interest binds to an analyte binding agent (such as, for example, an antibody to the analyte, or a receptor for the analyte), and the analyte and the analyte binding agent are thus referred to as "binding partners."

The sample to be analyzed for the presence of analyte is a blood sample, such as a serum sample or plasma sample. Blood plasma is the liquid component of blood in which the blood cells are suspended. A simple way to separate plasma from blood cells in a blood sample is by centrifugation. Serum refers to blood plasma in which clotting factors have been removed naturally by allowing the blood to clot prior to isolating the liquid component. Plasma samples can be obtained from blood tubes which contain anticoagulants such as sodium heparin, sodium citrate, sodium fluoride, and potassium oxalate or potassium EDTA (ethylenediamine tetraacetic acid). In the case of a plasma sample, the plasma can be obtained using an anticoagulant.

In many immunoassays, the analyte binding agent is an antibody. Such antibodies are often provided in a diluent such as buffer. The antibody may be of any immunoglobulin class, including, for example, IgG or IgM. The antibody may be a monoclonal antibody or a polyclonal antibody. Analytes of interest present in a sample can bind to immobilized capture antibodies, and then labeled antibody or antibodies in turn bind to the captured analyte. The label may be any known in the art, and include, for example, horseradish peroxidase and alkaline phosphatase. A detected signal is then indicative of an amount of analyte present in a sample. The method of detection can depend upon the type of label chosen and can include calorimetric, fluorometric, or chemiluminescent methods.

Prevention of false positive results can be a result of preventing a non-specific binding interaction.

Disclosed methods, compositions, and systems can be used for the in vitro qualitative detection of immunoglobulin G antibody to hepatitis C virus (anti-HCV) in human serum and/or plasma. In some embodiments, the detection is using VITROS ECi/ECiQ/3600 Immunodiagnostic Systems or VITROS 5600/XT 7600 Integrated Systems.

Disclosed methods, compositions and systems can be used in lateral flow assays, such as on slides or dry slides. The blocking agent(s) can be added to the slide before or during analysis. The blocking agent can be added as a coating in a channel or well within or on a slide.

The methods, compositions, and systems can be used in point of care systems wherein the analysis of human serum and/or plasma is desired.

The methods, compositions, and systems can be used in automated analyzers.

The blocking agent can be added to a lateral flow assay slide or chip as needed in order to run a sample.

Assay results, in conjunction with other laboratory results and clinical information, can be used to provide evidence of infection with hepatitis C virus in persons with signs or symptoms of hepatitis and in persons at risk for hepatitis C infection. Further, the assays as described herein may be used to screen for hepatitis C infection in pregnant women to identify neonates who are at high risk of acquiring HCV during the prenatal period.

The hepatitis C virus (HCV) is now known to be a causative agent for most, if not all, blood-borne non-A, non-B hepatitis (NANBH). Studies indicate that HCV is transmitted through contaminated blood and blood products, through blood transfusions or through other close, personal contacts. The presence of anti-HCV indicates that an individual may have been infected with HCV and may be capable of transmitting HCV infection. Three recombinant hepatitis C virus encoded antigens (c22-3, c200 and NS5) can be used to detect HCV. The recombinant protein c22-3 is encoded by the putative core region of the HCV genome. HCV recombinant protein c200 is encoded by the putative NS3 and NS4 regions of the HCV genome. The c200 protein contains the c33c protein sequence which is genetically linked to the c100-3 protein sequence. Studies have indicated that antibodies which develop after infection with HCV are often reactive with c22-3 and/or c33c. HCV recombinant protein NS5 is encoded by the putative NS5 region of the HCV genome. A significant proportion of persons infected with HCV develop antibodies to NS5. The host organism for all three HCV recombinant antigens is S. *cerevisiae* (yeast).

The compositions used for detection can include three recombinant hepatitis C virus encoded antigens. One or two can be used. C22-3, c200 and NS5 can be used. C22-3 and c200, or c22-3 and NS5, or c200 and NS5 can be used.

Hepatitis C (HCV) often affects the liver and is a major concern for both patient care and the world's blood supply. This infection produces HCV antibodies in a patient's blood that can be captured and detected by diagnostic immunoassays. These immunoassays play a key role in identifying infected patients and blood samples. While these assays are designed to be very sensitive and specific, false positive results are a reality. A false positive result occurs when a diagnostic tool such as an immunoassay gives a positive result when there is, in fact, no HCV antibodies present in a test sample. These false positive results can occur for a variety of reasons including, but not limited to, non-specific binding.

Non-specific binding occurs when a compound in a sample is captured and detected by an assay when that compound is not the target analyte (i.e. not the HCV antibody). A blocking agent as described herein can be included in assay reagent formulations to minimize these false positives.

The blocking agents described can reduce a rate of false positive results detected in samples and/or over an entire patient population.

The blocking agent can be added to a reagent mixture or added as a reagent during an assay.

A blocking agent can include a glycosaminoglycan, such as but not limited to heparin, chondroitin, dermatan, keratin, and hyaluronic acid. The glycosaminoglycan can be heparin. That heparin can be coordinated with a metal, such as a metal ion. That metal can be an alkali metal such as a Group I element. Metals can include lithium, sodium, potassium, rubidium, francium, or cesium.

The blocking agent can include lithium. In the invention, the blocking agent is lithium heparin.

Lithium heparin is a negatively charged polysaccharide polymer classified as a glycosoaminoglycan and is produced and stored within mammalian tissues.

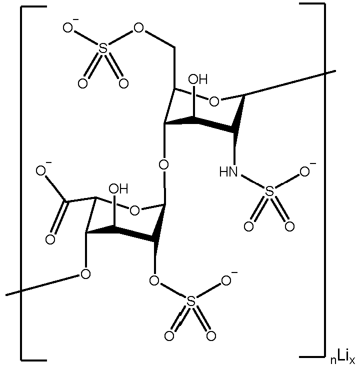

The heparin as described herein can have a chain length that results in reduced false positives. In some embodiments, n can be 1-100, 1-1,000, or 1-10,000.

Heparin has been utilized in medicine as an anticoagulant as it interacts with several cofactors within the human coagulation process. Lithium heparin is the lithium salt of heparin.

The blocking agent is provided in an amount sufficient to reduce non-specific binding in an assay of a sample for the analyte. When the blocking agent is lithium heparin, the amount is preferably from about 0.25 mg/mL to about 4 mg/mL (equivalent to about 0.025% to about 0.4%).

In some embodiments, an immunometric technique is used. Such a technique involves a two-stage reaction. In the first stage, HCV antibody present in the sample binds with HCV recombinant antigens coated on the wells. Unbound sample is removed by washing. In the second stage, horseradish peroxidase (HRP)-labeled antibody conjugate (mouse monoclonal anti-human IgG) binds to any human IgG captured on the well in the first stage. Unbound conjugate is removed by washing.

The bound HRP conjugate is measured by a luminescent reaction. A reagent containing luminogenic substrates (a luminol derivative and a peracid salt) and an electron transfer agent, is added to the wells. The HRP in the bound conjugate catalyzes the oxidation of the luminol derivative, producing light. The electron transfer agent (a substituted acetanilide) increases the level of light produced and prolongs its emission. The light signals are read by the system. The amount of HRP conjugate bound is indicative of the level of anti-HCV present in the sample.

A reagent composition can include a blocking agent as described herein in combination with diagnostic regents in a buffer. Those diagnostic reagents may be immunodiagnostic reagents.

A reagent composition can include a blocking agent as described herein, a labeled protein, and antigens in buffer.

A reagent composition can include a blocking agent as described herein, a HRP-labeled protein, and antigens in buffer.

A reagent composition can include a blocking agent as described herein, a HRP-labeled protein, an antimicrobial agent, and antigens in buffer.

A reagent composition can include a blocking agent as described herein, a HRP-labeled protein, and HCV encoded antigens in buffer.

A reagent composition can include a blocking agent as described herein, a HRP-labeled protein, an antimicrobial agent, and HCV encoded antigens in buffer.

A reagent composition can include a blocking agent as described herein, a HRP-labeled IgG, and HCV encoded antigens in buffer.

A reagent composition can include a blocking agent as described herein, a HRP-labeled IgG, an antimicrobial agent, and HCV encoded antigens in buffer.

A reagent composition can include lithium heparin, a labeled protein, and antigens in buffer.

A reagent composition can include lithium heparin, a HRP-labeled protein, and antigens in buffer.

A reagent composition can include lithium heparin, a HRP-labeled protein, an antimicrobial agent, and antigens in buffer.

A reagent composition can include lithium heparin, a HRP-labeled protein, and HCV encoded antigens in buffer.

A reagent composition can include lithium heparin, a HRP-labeled protein, an antimicrobial agent, and HCV encoded antigens in buffer.

A reagent composition can include lithium heparin, a HRP-labeled IgG, and HCV encoded antigens in buffer.

A reagent composition can include lithium heparin, a HRP-labeled IgG, an antimicrobial agent, and HCV encoded antigens in buffer.

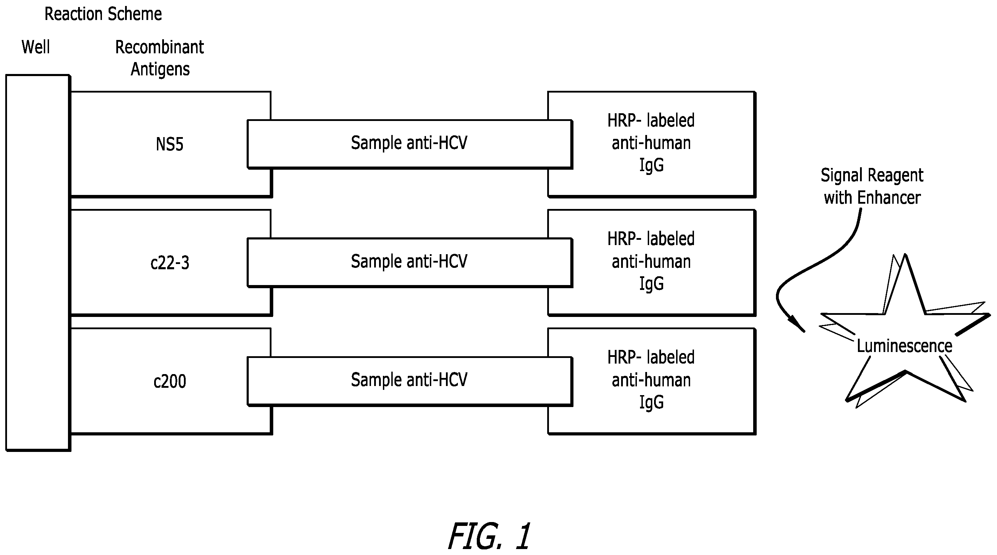

As described, three recombinant hepatitis C virus encoded antigens (c22-3, c200 and NS5) can be used to detect HCV. When used, a reaction scheme similar to that in FIG. 1 is used, wherein each of the three antigens are measured using HRP labeled anti-human IgG.

Reagents used to detect HCV can include Hepatitis C virus recombinant antigens (NS5, c22-3, c200) derived from yeast (S. cerevisiae), reagent (buffer with 2-chloroacetamide anti-microbial agent), conjugate reagent (HRP-mouse monoclonal anti-human IgG, 1.04 ng/well) in buffer with anti-microbial agent (1% ProClin 300 w/w), and lithium heparin.

Reagents used to detect HCV can include Hepatitis C virus recombinant antigens (NS5, c22-3, c200) derived from yeast (S. cerevisiae), 18.2 mL assay reagent (buffer with 2-chloroacetamide anti-microbial agent), 20.6 mL conjugate reagent (HRP-mouse monoclonal anti-human IgG, 1.04 ng/well) in buffer with anti-microbial agent (1% ProClin 300 w/w), and lithium heparin.

The Hepatitis C virus recombinant antigens (NS5, c22-3, c200) can be coated on wells, such as wells in a 100 well plate.

The reagent packs including the blocking agent are kept refrigerated in order to preserve stability.

Inclusion of heparin, in particular lithium heparin, in the assay reagent for an HCV immunoassay can markedly reduce the occurrence of false positives. Heparin may bind to a compound within a serum or plasma sample that, when unbound, can be the cause of a non-specific binding events, leading to false positive assay results. The addition of heparin, in particular lithium heparin, into the formulation of an immunodiagnostic assay's reagents is able to reduce the incidence rate of false positives, increasing the assay's overall specificity.

In some embodiments, including lithium heparin in an assay can reduce false positive rates by greater than about 70%, greater than about 80%, greater than about 85%, greater than about 86%, greater than about 90%, or greater than about 95%.

In some assays, lithium heparin does not impact the sensitivity of assays.

In some assays, lithium heparin does not affect the assay's ability to detect true positive samples.

Lithium heparin can increase the specificity of an assay. In some embodiments, specificity can increase by greater than about 0.05%, greater than about 0.1 %, greater than about 0.2%, greater than about 0.3%, greater than about 0.4%, or greater than about 0.5%.

Also disclosed for information are methods of detecting an analyte in a sample comprising combining a sample to be analyzed for the presence of an analyte with a blocking agent and an analyte binding agent, so as to form a complex of any analyte present in the sample and the analyte binding agent, wherein the blocking agent reduces non-specific binding in the method; and detecting the resulting complex so as to detect the analyte. The sample may be combined with the blocking agent and the resulting sample is then combined with the analyte binding agent. The sample may be combined with the analyte binding agent and the resulting sample is then combined with the blocking agent. The analyte binding agent is provided as an immunoassay reagent comprising the analyte binding agent in a diluent and the blocking agent, and the immunoassay reagent is combined with the sample.

### Example 1

Lithium heparin is added to an assay reagent of a VITROS Immunodiagnostic Product HCV assay at a concentration of 0.04 to 0.10% w/v. The assay is then used to analyze a series of 112 samples known to give a false positive result when using this assay. These samples previously tested as positive on the VITROS HCV assay, but are then proven to be negative by immuno-blot and/or nucleic acid testing (NAT). Of these 112 samples, only 16 continue to give a false positive result once lithium heparin is added to the assay's reagents.

FIG. 1 illustrates results of this study. The x-axis represents sample ID number and the y-axis represents the signal-to-cutoff value (S/C). Any sample with a result > 1.0 S/C is considered positive in this assay, the VITROS HCV assay. The control data set are the results given on the unmodified assay, while the lithium heparin data points are the result of the same sample when the assay contains the lithium heparin additive in the assay reagent. This suggests that addition of lithium heparin reduces the false positive rate.

### Example 2

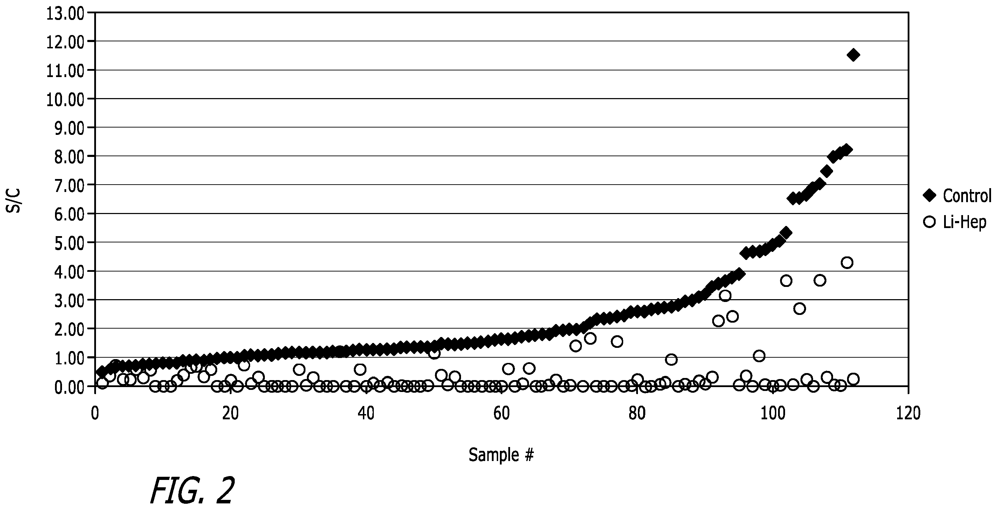

Lithium heparin does not significantly impact the sensitivity of assays. In other words, it does not affect the assay's ability to detect true positive samples. Four seroconversion panel members (samples collected from a recently infected donor during the development an initial immune response) near the 1.0 S/C cutoff for a positive result are investigated. The results are illustrated in FIG. 2.

The left bar result is that given by the on-market VITROS HCV assay, and the right bar result is this assay with the lithium heparin additive. The addition of heparin did not change the classification of any of these four positive samples.

### Example 3

The addition of lithium heparin can increase the specificity of an assay. One thousand presumed negative patient samples are run on an on-market VITROS HCV assay and then again on the same assay with the lithium heparin additive. Specificity increases from 99.80% to 99.90%. This assay and similar diagnostic products are used to screen millions of patients and blood samples each year. This reduction in false positives has the potential to correct tens of thousands of results annually.

Unless otherwise indicated, all numbers expressing quantities of ingredients, properties such as molecular weight, reaction conditions, and so forth used in the specification and claims are to be understood as being modified in all instances by the term "about." Accordingly, unless indicated to the contrary, the numerical parameters set forth in the specification and attached claims are approximations that may vary depending upon the desired properties sought to be obtained by the present invention. At the very least, and not as an attempt to limit the application of the doctrine of equivalents to the scope of the claims, each numerical parameter should at least be construed in light of the number of reported significant digits and by applying ordinary rounding techniques. Notwithstanding that the numerical ranges and parameters setting forth the broad scope of the invention are approximations, the numerical values set forth in the specific examples are reported as precisely as possible. Any numerical value, however, inherently contains certain errors necessarily resulting from the standard deviation found in their respective testing measurements.

The terms "a," "an," "the" and similar referents used in the context of describing the invention (especially in the context of the following claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein is intended merely to better illuminate the invention and does not pose a limitation on the scope of the invention otherwise claimed. No language in the specification should be construed as indicating any non-claimed element essential to the practice of the invention.

Groupings of alternative elements or embodiments of the invention disclosed herein are not to be construed as limitations. Each group member may be referred to and claimed individually or in any combination with other members of the group or other elements found herein. It is anticipated that one or more members of a group may be included in, or deleted from, a group for reasons of convenience and/or patentability. When any such inclusion or deletion occurs, the specification is deemed to contain the group as modified thus fulfilling the written description of all Markush groups used in the appended claims.

Certain embodiments of this invention are described herein, including the best mode known to the inventors for carrying out the invention. Of course, variations on these described embodiments will become apparent to those of ordinary skill in the art upon reading the foregoing description.

## Claims

1. Use of lithium heparin as a blocking reagent to reduce a rate of false positive results in an immunoassay for detection of hepatitis C antibodies in a blood sample.

2. The use of claim 1, wherein the amount of lithium heparin is from 0.25 mg/mL to 4 mg/mL.

3. The use of claim 1 or 2, wherein the blood sample is a serum sample.

4. The use of claim 1 or 2, wherein the blood sample is a plasma sample.

5. The use of claim 4, wherein the plasma sample contains ethylenediamine tetraacetic acid.

6. The use of claim 1, further including an anti-microbial agent.

7. The use of claim 1, wherein the rate of false positive results is reduced by greater than 70%.

8. The use of claim 1, wherein lithium heparin does not affect the detection of hepatitis C antibodies.

9. The use of claim 1, wherein specificity of the immunoassay increases by greater than 0.05%.

10. The use of claim 1, wherein the false positive results occur due to non-specific binding.

11. The use of claim 1, wherein the immunoassay is used in lateral flow assays.

12. The use of claim 1, wherein the immunoassay is used in point of care systems.

13. The use of claim 1, wherein the immunoassay is used in automated analyzers.

14. The use of claim 1, wherein the immunoassay is used in persons at risk for hepatitis C infection.

15. The use of claim 1, wherein the immunoassay is used in pregnant women.

## Patentansprüche

1. Verwendung von Lithiumheparin als Blockierungsreagens zur Verringerung einer Rate falsch positiver Ergebnisse in einem Immunoassay zum Nachweis von Hepatitis-C-Antikörpern in einer Blutprobe.

2. Verwendung gemäß Anspruch 1, wobei die Menge an Lithiumheparin 0,25 mg/ml bis 4 mg/ml beträgt.

3. Verwendung gemäß Anspruch 1 oder 2, wobei die Blutprobe eine Serumprobe ist.

4. Verwendung gemäß Anspruch 1 oder 2, wobei die Blutprobe eine Plasmaprobe ist.

5. Verwendung gemäß Anspruch 4, wobei die Plasmaprobe Ethylendiamintetraessigsäure enthält.

6. Verwendung gemäß Anspruch 1, die ferner ein antimikrobielles Mittel umfasst.

7. Verwendung gemäß Anspruch 1, wobei die Rate falsch positiver Ergebnisse um mehr als 70 % verringert wird.

8. Verwendung gemäß Anspruch 1, wobei Lithiumheparin den Nachweis von Hepatitis-C-Antikörpern nicht beeinträchtigt.

9. Verwendung gemäß Anspruch 1, wobei die Spezifität des Immunoassays um mehr als 0,05 % zunimmt.

10. Verwendung gemäß Anspruch 1, wobei die falsch positiven Ergebnisse aufgrund nichtspezifischer Bindung auftreten.

11. Verwendung gemäß Anspruch 1, wobei der Immunoassay in Lateral-Flow-Assays verwendet wird.

12. Verwendung gemäß Anspruch 1, wobei der Immunoassay in Point-of-Care-Systemen verwendet wird.

13. Verwendung gemäß Anspruch 1, wobei der Immunoassay in automatisierten Analysatoren verwendet wird.

14. Verwendung gemäß Anspruch 1, wobei der Immunoassay bei Personen mit Risiko für eine Hepatitis-C-Infektion verwendet wird.

15. Verwendung gemäß Anspruch 1, wobei der Immunoassay bei schwangeren Frauen verwendet wird.

## Revendications

1. Utilisation d'héparine de lithium comme réactif de blocage afin de réduire un taux de résultats faux positifs dans un dosage immunologique pour la détection d'anticorps contre l'hépatite C dans un échantillon de sang.

2. L'utilisation de la revendication 1, dans laquelle la quantité d'héparine de lithium va de 0,25 mg/ml à 4 mg/ml.

3. L'utilisation de la revendication 1 ou de la revendication 2, dans laquelle l'échantillon de sang est un échantillon de sérum.

4. L'utilisation de la revendication 1 ou de la revendication 2, dans laquelle l'échantillon de sang est un échantillon de plasma.

5. L'utilisation de la revendication 4, dans laquelle l'échantillon de plasma contient de l'acide éthylènediaminetétraacétique.

6. L'utilisation de la revendication 1, incluant en sus un agent antimicrobien.

7. L'utilisation de la revendication 1, dans laquelle le taux de résultats faux positifs est réduit de plus de 70 %.

8. L'utilisation de la revendication 1, dans laquelle l'héparine de lithium n'affecte pas la détection d'anticorps contre l'hépatite C.

9. L'utilisation de la revendication 1, dans laquelle la spécificité du dosage immunologique augmente de plus de 0,05 %.

10. L'utilisation de la revendication 1, dans laquelle les résultats faux positifs se produisent en raison d'une liaison non spécifique.

11. L'utilisation de la revendication 1, dans laquelle le dosage immunologique est utilisé dans des dosages à flux latéral.

12. L'utilisation de la revendication 1, dans laquelle le dosage immunologique est utilisé dans des systèmes de biologie médicale délocalisée.

13. L'utilisation de la revendication 1, dans laquelle le dosage immunologique est utilisé dans des analyseurs automatisés.

14. L'utilisation de la revendication 1, dans laquelle le dosage immunologique est utilisé chez des personnes à risque d'infection par l'hépatite C.

15. L'utilisation de la revendication 1, dans laquelle le dosage immunologique est utilisé chez des femmes enceintes.
